# FASCICULE DE BREVET EUROPEEN

(11) **EP 2 763 947 B1**
(45) Date de publication et mention de la délivrance du brevet: **10.02.2016**
(21) Numéro de dépôt: 12768852.1
(22) Date de dépôt: 05.10.2012
(51) Int. Cl.: C07C 45/64, C07C 47/575

(54) **PROCÉDÉ DE PRÉPARATION D'UN DÉRIVÉ DE VANILLINE**
VERFAHREN ZUR HERSTELLUNG EINES VANILLINDERIVATS
METHOD FOR PREPARING A VANILLIN DERIVATIVE

(30) Priorité: 06.10.2011 FR 1159007
(43) Date de publication de la demande: 13.08.2014
(73) Titulaire: Rhodia Operations, 75009 Paris (FR)
(72) Inventeur: GAREL, Laurent, F-69003 Lyon (FR); VIBERT, Martine, F-69006 Lyon (FR); COCHENNEC, Corine, F-38500 Voiron (FR); METZ, François, F-69540 Irigny (FR)
(74) Mandataire: Ridray, Annabelle
(86) Numéro de dépôt international: PCT/EP2012/069732
(87) Numéro de publication internationale: WO 2013/050537

(56) Documents cités:
- WO-A1-96/37452
- Hui Xu ET AL: "Reactivity of Lignin Diphenylmethane Model Dimers l. Nitrobenzene Oxidation", Holzforschung, 1 janvier 1998 (1998-01-01), pages 51-56, XP055026616, Extrait de l'Internet: URL:http://www.degruyter.com/view/j/hfsg.1 998.52.issue-1/hfsg.1998.52.1.51/hfsg.1998 .52.1.51.xml?format=INT [extrait le 2012-05-09]
- BERND SCHWARZ ET AL: "Identification of Novel Orosensory Active Molecules in Cured Vanilla Beans (Vanilla planifolia)", JOURNAL OF AGRICULTURAL AND FOOD CHEMISTRY, vol. 57, no. 9, 13 mai 2009 (2009-05-13), pages 3729-3737, XP055026540, ISSN: 0021-8561, DOI: 10.1021/jf900086m

## Description

La présente invention a pour objet un procédé de préparation d'un dérivé de vanilline, de type dimère, nommé 3-(4-hydroxy-3-méthoxybenzyl)-4-hydroxy-5-méthoxybenzaldéhyde.

La vanilline est un produit largement utilisé dans de nombreux domaines d'application en tant qu'arôme et/ou parfum.

Ainsi, la vanilline se trouve abondamment consommée dans l'industrie alimentaire et animale mais elle a aussi des applications dans d'autres domaines tels que par exemple, la pharmacie ou la parfumerie.

Les procédés actuellement utilisés pour préparer la vanilline peuvent conduire à des sous-produits à l'état de traces, parmi lesquelles notamment le 3-(4-hydroxy-3-méthoxybenzyl)-4-hydroxy-5-méthoxybenzaldéhyde.

Or, ce sous-produit de formule 3-(4-hydroxy-3-méthoxybenzyl)-4-hydroxy-5-méthoxybenzaldéhyde présente des propriétés sensorielles intéressantes.

Toutefois, il n'existe pas à ce jour de procédé de préparation du 3-(4-hydroxy-3-méthoxybenzyl)-4-hydroxy-5-méthoxybenzaldéhyde en tant que tel avec un rendement satisfaisant.

Hui Xu dans Holzforschung,1998, vol.52, (p.51-56) divulgue un procédé en 2 étapes par condensation alkaline entre l'alcool vanillique et le créosol (2-méthoxy-4-methylphénol) suivie d'une oxydation du methyl en groupement aldéhyde donnant le produit désiré avec un rendement assez faible de 29%.

La présente invention a pour but de fournir un nouveau procédé de préparation du 3-(4-hydroxy-3-méthoxybenzyl)-4-hydroxy-5-méthoxybenzaldéhyde.

La présente invention a pour but de fournir un procédé permettant d'obtenir le 3-(4-hydroxy-3-méthoxybenzyl)-4-hydroxy-5-méthoxybenzaldéhyde avec un rendement satisfaisant.

Ainsi, la présente invention concerne un procédé de préparation d'un composé de formule (I) suivante : 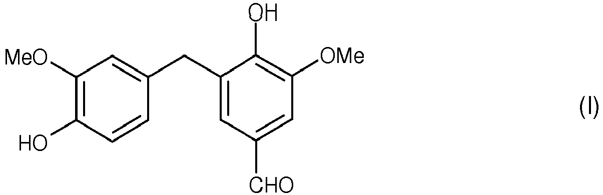 comprenant la réaction de la vanilline et l'alcool vanillique en présence d'une base.

La vanilline ou 4-hydroxy-3-méthoxybenzaldéhyde est une molécule bien connue de l'état de la technique répondant à la formule suivante :

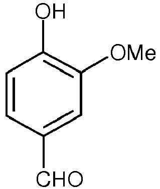

L'alcool vanillique ou alcool 4-hydroxy-3-méthoxybenzylique est une molécule bien connue de l'état de la technique répondant à la formule suivante :

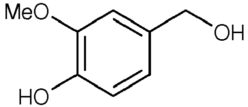

La présente invention est basée sur une réaction d'addition de l'alcool vanillique (VOH) sur la vanilline (VA) en présence d'une base.

Il a été constaté de façon surprenante que la fonction aldéhyde de la vanilline reste inchangée. Dans le cadre du procédé de l'invention, cette fonction ne réagit pas avec la fonction alcool de l'alcool vanillique.

Selon un mode de réalisation, la base est choisie dans le groupe constitué par les bases minérales, les bases organiques, les bases hétérogènes minérales et leurs mélanges.

A titre de bases organiques, on peut notamment citer les amines tertiaires, par exemple la triméthylamine, la triéthylamine, la tripropylamine, la tributylamine, la N,N-diisopropyléthylamine, la N-méthylmorpholine, la N-éthylmorpholine, la N-propylmorpholine et la 1-méthylpyrrolidone. On peut encore utiliser des bases anioniques azotées, par exemple les sels, notamment alcalins ou alcalino-terreux, d'amines, silylées ou non, ainsi que des silylamines. Les disilylamines salifiées et notamment les sels, en particulier alcalins ou alcalino-terreux, de l'hexaméthyldisilazane (HMDZ) sont également appropriés.

A titre de bases hétérogènes minérales, on peut notamment citer en particulier le dioxyde de magnésium MgO₂.

De préférence, l'étape de réaction du procédé est effectuée en présence d'une base minérale.

Selon un mode de réalisation, la base est choisie dans le groupe constitué des hydroxydes de métaux alcalins, des hydroxydes de métaux alcalino-terreux, des bicarbonates de métaux alcalins, des bicarbonates de métaux alcalino-terreux, des hydrogénocarbonates de métaux alcalins, des hydrogénocarbonates de métaux alcalino-terreux, des phosphates de métaux alcalins, des phosphates de métaux alcalino-terreux, des hydrogénophosphates de métaux alcalins, des hydrogénophosphates de métaux alcalino-terreux, et leurs mélanges.

A titre de bases minérales préférées, on peut citer LiOH, CsOH, NaOH, KOH, Na₂CO₃ ou NaHCO₃. Préférentiellement, on utilise Na₂CO₃ à titre de base.

Selon le procédé de l'invention, l'étape de réaction susmentionnée peut être effectuée en présence d'un solvant.

A titre de solvant, on peut citer les solvants aqueux et organiques, et leurs mélanges.

De préférence, le procédé de l'invention est effectué en milieu aqueux : le procédé selon la présente invention est effectué de préférence en présence d'eau à titre de solvant.

Dans le cadre du procédé de l'invention, lorsque la base utilisée est une base organique, la réaction est effectuée en présence d'un solvant organique. On choisit un solvant qui soit inerte dans les conditions réactionnelles. Ledit solvant est par exemple choisi parmi les solvants organiques apolaires tels que les hydrocarbures aliphatiques (préférentiellement les alcanes de formule CₙH₂ₙ₊₂ avec n= 6 à 14), les hydrocarbures cycloaliphatiques tel que le cyclohexane, les hydrocarbures aromatiques (préférentiellement le benzène, le toluène, les xylènes, l'éthylbenzène, les diéthylbenzènes, les triméthylbenzènes, le cumène), les hydrocarbures halogénés aliphatiques ou aromatiques, en particulier les hydrocarbures perchlorés (préférentiellement le trichlorométhane, le tétrachlorométhane) et les hydrocarbures partiellement chlorés (tels que dichlorométhane, tétrachloroéthane, monochlorobenzène, les dichlorobenzènes). Ledit solvant organique est encore avantageusement choisi parmi les solvants organiques aprotiques polaires, par exemple le diméthylformamide le diméthylacétamide. Il peut encore être très préférentiellement choisi parmi les solvants protiques polaires tels que les alcools, en particulier l'éthanol.

Selon un mode de réalisation, le procédé de l'invention est effectué en milieu biphasique, c'est-à-dire en présence d'eau et d'un solvant organique, préférentiellement d'un solvant organique non soluble. Selon ce mode de réalisation, le rapport entre le volume de solvant organique et le volume d'eau est compris de 0,1 à 10, et de préférence de 0,5 à 1,5.

La réaction du procédé de l'invention est effectuée à une température comprise de 0 °C à 100 °C, de préférence de 30 °C à 80 °C.

De préférence, dans le cadre de la présente invention, la réaction est effectuée à un pH compris de 7 à 14, de préférence de 8 à 10.

La présente invention concerne également un procédé de préparation tel que défini ci-dessus, dans lequel le rapport entre le nombre de moles de vanilline et le nombre de moles d'alcool vanillique ([VA/VOH]ₘₒₗ) est compris de 0,5 à 20, de préférence de 1 à 15, préférentiellement de 2 à 5, et notamment de 3 à 5.

Selon un mode de réalisation avantageux du procédé de l'invention, le rapport entre le nombre de moles de base et la somme du nombre de moles de vanilline et du nombre de moles d'alcool vanillique est compris de 0,1 à 10, de préférence de 0,5 à 6, et préférentiellement de 0,7 à 2. Préférentiellement, ce ratio est voisin de 1 et très préférentiellement égal à 1.

Selon un mode de réalisation, dans le cadre du procédé de l'invention, lorsque la réaction est effectuée en présence d'eau, le rapport entre la masse de vanilline et d'alcool vanillique et la masse d'eau est compris de 0,05 à 0,5, de préférence de 0,1 à 0,3.

Le procédé de l'invention consiste en une réaction entre la vanilline et l'alcool vanillique en présence d'une base. Ces différents réactifs, à savoir la vanilline, l'alcool vanillique et la base peuvent être mis en présence par tout moyen connu de l'homme du métier.

De même, ces réactifs peuvent être introduits dans un ordre quelconque.

Le solvant peut également être introduit dans un ordre quelconque par rapport aux réactifs.

Les réactifs ainsi que le solvant peuvent également être introduits en une fois ou en plusieurs fois, c'est-à-dire de façon fractionnée.

Selon un mode de réalisation, on peut ajouter le solvant, notamment l'eau, à un mélange comprenant la base et la vanilline puis ajouter par la suite l'alcool.

Selon un autre mode de réalisation, on peut ajouter la base en plusieurs fois. Par exemple, on peut ajouter le solvant, notamment l'eau, à un mélange comprenant la base et la vanilline puis ajouter par la suite, de façon simultanée, l'alcool et la base.

A l'issue du procédé de l'invention, on obtient une solution liquide comprenant le 3-(4-hydroxy-3-méthoxybenzyl)-4-hydroxy-5-méthoxybenzaldéhyde.

Ce composé peut ensuite être soumis à des étapes ultérieures classiques de séparation, notamment par distillation.

Le composé obtenu par le procédé de l'invention est avantageusement utilisé dans des compositions alimentaires, c'est-à-dire des compositions destinées à être consommées par l'homme.

Il présente un effet de rondeur en bouche et un tel effet est recherché de manière à pallier le caractère insipide de nombreux produits alimentaires présentant de faibles taux de matières grasses et de sucres. Cet effet de rondeur en bouche procure au consommateur une sensation agréable en bouche, notamment par un caractère crémeux, un caractère de matières grasses du lait et/ou un caractère sucré.

Il peut par exemple être utilisé dans une combinaison d'arômes, en particulier d'arômes alimentaires, comprenant des composés tels que la vanilline ou l'éthylvanilline. Le composé obtenu selon le procédé de l'invention est avantageusement incorporé dans des compositions alimentaires se présentant sous forme liquide, notamment les boissons, le lait et les soupes, sous forme semi-solide ou solide, notamment les yaourts, les margarines, les desserts instantanés et les crèmes glacées, les biscuits et gâteaux, la confiserie et la chocolaterie.

Ainsi, le 3-(4-hydroxy-3-méthoxybenzyl)-4-hydroxy-5-méthoxybenzaldéhyde est avantageusement utilisé pour conférer ou renforcer certaines sensations de goût.

On donne ci-après des exemples illustrant la présente invention, sans caractère limitatif.

### EXEMPLES

Dans les exemples, les pourcentages mentionnés sont exprimés en poids.

Les réactifs utilisés, à savoir la vanilline, l'alcool vanillique et le carbonate de sodium Na₂CO₃ sont introduits de façon simultanée.

Les réactions sont effectuées en présence d'eau.

Les tableaux ci-dessous indiquent les conditions opératoires mises en oeuvre ainsi que les quantités des différents réactifs engagées.

| | VA (mmol) | VOH (mmol) | ratio molaire VA/VOH | base Na₂CO₃ (mmol) | T (°C) | base (mmol) / VA + VOH (mmol) | 1 (mmol) |
|---|---|---|---|---|---|---|---|
| Ex.1 | 6,71 | 5,19 | 1,29 | 12,29 | 35 | 1,03 | 0,89 |
| Ex.2 | 12,10 | 5,10 | 2,37 | 17,08 | 40 | 0,99 | 1,19 |

| | VA (g) | VOH (g) | eau (g) | VA (g) + VOH (g) / eau (g) | TT VA (%) | TT VOH (%) | RT 1 (%) |
|---|---|---|---|---|---|---|---|
| Ex.1 | 1,02 | 0,8 | 20,39 | 0,09 | 20 | 89 | 66 |
| Ex.2 | 1,84 | 0,78 | 21,00 | 0,12 | 13 | 62 | 76 |

| | | | | | | | |
|---|---|---|---|---|---|---|---|
| VA : vanilline VOH : alcool vanillique 1 : 3-(4-hydroxy-3-méthoxybenzyl)-4-hydroxy-5-méthoxybenzaldéhyde TT : taux de transformation (représente la quantité de réactif transformé par rapport à la quantité de réactif introduit) RT : rendement (rapport entre le nombre de moles de composé 1 obtenu et le nombre de moles de vanilline transformée) | | | | | | | |

Ainsi, le procédé de l'invention permet d'obtenir le composé 3-(4-hydroxy-3-méthoxybenzyl)-4-hydroxy-5-méthoxybenzaldéhyde avec un rendement très satisfaisant.

## Revendications

1. Procédé de préparation d'un composé de formule (I) suivante : 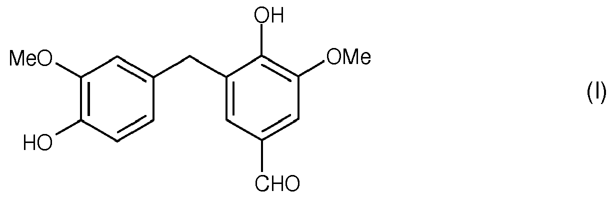 comprenant la réaction de la vanilline et l'alcool vanillique en présence d'une base.

2. Procédé selon la revendication 1, dans lequel la base est choisie dans le groupe constitué des bases minérales, des bases organiques, des bases hétérogènes minérales et de leurs mélanges.

3. Procédé selon la revendication 2, dans lequel la base est choisie dans le groupe constitué des hydroxydes de métaux alcalins, des hydroxydes de métaux alcalino-terreux, des bicarbonates de métaux alcalins, des bicarbonates de métaux alcalino-terreux, des hydrogénocarbonates de métaux alcalins, des hydrogénocarbonates de métaux alcalino-terreux, des phosphates de métaux alcalins, des phosphates de métaux alcalino-terreux, des hydrogénophosphates de métaux alcalins, des hydrogénophosphates de métaux alcalino-terreux, et de leurs mélanges.

4. Procédé selon l'une quelconque des revendications 1 à 3, dans lequel la réaction est effectuée en présence d'un solvant.

5. Procédé selon la revendication 4, dans lequel le solvant est choisi parmi les solvants aqueux et organiques, et leurs mélanges.

6. Procédé selon la revendication 4 ou la revendication 5, dans lequel le solvant est l'eau.

7. Procédé selon l'une quelconque des revendications 1 à 6, dans lequel la réaction est effectuée à une température comprise de 0°C à 100°C.

8. Procédé selon l'une quelconque des revendications 1 à 7, dans lequel la réaction est effectuée à un pH compris de 7 à 14.

9. Procédé selon l'une quelconque des revendications 1 à 8, dans lequel le rapport entre le nombre de moles de vanilline et le nombre de moles d'alcool vanillique est compris de 0,5 à 20.

10. Procédé selon l'une quelconque des revendications 1 à 9, dans lequel le rapport entre le nombre de moles de base et la somme du nombre de moles de vanilline et du nombre de moles d'alcool vanillique est compris de 0,1 à 10.

11. Procédé selon l'une quelconque des revendications 6 à 10, dans lequel le rapport entre la masse de vanilline et d'alcool vanillique et la masse d'eau est compris de 0,05 à 0,5.

## Patentansprüche

1. Verfahren zur Herstellung einer Verbindung der folgenden Formel (I): 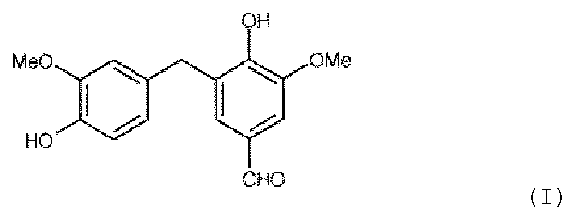 umfassend die Umsetzung von Vanillin und Vanillylalkohol in Gegenwart einer Base.

2. Verfahren nach Anspruch 1, wobei die Base aus der Gruppe ausgewählt wird, die aus anorganischen Basen, organischen Basen, anorganischen heterogenen Basen und deren Mischungen besteht.

3. Verfahren nach Anspruch 2, wobei die Base aus der Gruppe ausgewählt wird, die aus Alkalimetallhydroxiden, Erdalkalimetallhydroxiden, Alkalimetallbicarbonaten, Erdalkalimetallbicarbonaten, Alkalimetallhydrogencarbonaten, Erdalkalimetallhydrogencarbonaten, Alkalimetallphosphaten, Erdalkalimetallphosphaten, Alkalimetallhydrogenphosphaten, Erdalkalimetallhydrogenphosphaten und deren Mischungen besteht.

4. Verfahren nach einem der Ansprüche 1 bis 3, wobei die Umsetzung in Gegenwart eines Lösungsmittels erfolgt.

5. Verfahren nach Anspruch 4, wobei das Lösungsmittel aus wässrigen und organischen Lösungsmitteln und deren Mischungen ausgewählt ist.

6. Verfahren nach Anspruch 4 oder Anspruch 5, wobei es sich bei dem Lösungsmittel um Wasser handelt.

7. Verfahren nach einem der Ansprüche 1 bis 6, wobei die Umsetzung bei einer Temperatur von 0°C bis 100°C erfolgt.

8. Verfahren nach einem der Ansprüche 1 bis 7, wobei die Umsetzung bei einem pH-Wert von 7 bis 14 erfolgt.

9. Verfahren nach einem der Ansprüche 1 bis 8, wobei das Verhältnis zwischen der Molzahl an Vanillin und der Molzahl an Vanillylalkohol 0,5 bis 20 beträgt.

10. Verfahren nach einem der Ansprüche 1 bis 9, wobei das Verhältnis zwischen der Molzahl an Base und der Summe der Molzahl an Vanillin und der Molzahl an Vanillylalkohol 0,1 bis 10 beträgt.

11. Verfahren nach einem der Ansprüche 6 bis 10, wobei das Verhältnis zwischen der Masse an Vanillin und an Vanillylalkohol und der Masse an Wasser 0,05 bis 0,5 beträgt.

## Claims

1. Process for preparing a compound of formula (I) below: 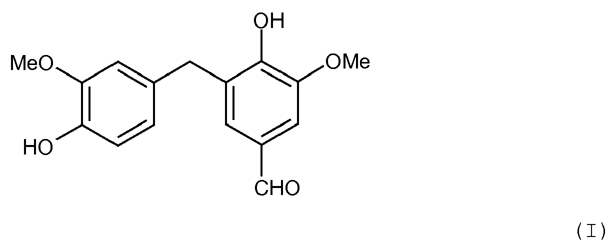 comprising the reaction of vanillin and vanillyl alcohol in the presence of a base.

2. Process according to Claim 1, wherein the base is selected from the group consisting of inorganic bases, organic bases, inorganic heterogeneous bases, and mixtures thereof.

3. Process according to Claim 2, wherein the base is selected from the group consisting of alkali metal hydroxides, alkaline-earth metal hydroxides, alkali metal bicarbonates, alkaline-earth metal bicarbonates, alkali metal hydrogen carbonates, alkaline-earth metal hydrogen carbonates, alkali metal phosphates, alkaline-earth metal phosphates, alkali metal hydrogen phosphates and alkaline-earth metal hydrogen phosphates, and mixtures thereof.

4. Process according to any one of Claims 1 to 3, wherein the reaction is carried out in the presence of a solvent.

5. Process according to Claim 4, wherein the solvent is selected from aqueous and organic solvents, and mixtures thereof.

6. Process according to Claim 4 or Claim 5, wherein the solvent is water.

7. Process according to any one of Claims 1 to 6, wherein the reaction is carried out at a temperature included from 0°C to 100°C.

8. Process according to any one of Claims 1 to 7, wherein the reaction is carried out at a pH included from 7 to 14.

9. Process according to any one of Claims 1 to 8, wherein the ratio between the number of moles of vanillin and the number of moles of vanillyl alcohol is included from 0.5 to 20.

10. Process according to any one of Claims 1 to 9, wherein the ratio between the number of moles of base and the sum of the number of moles of vanillin and of the number of moles of vanillyl alcohol is included from 0.1 to 10.

11. Process according to any one of Claims 6 to 10, wherein the ratio between the weight of vanillin and of vanillyl alcohol and the weight of water is included from 0.05 to 0.5.
